(19) Europäisches Patentamt
European Patent Office
Office européen des brevets

(11) **EP 4 321 243 A1**

(12) **EUROPEAN PATENT APPLICATION**
published in accordance with Art. 153(4) EPC

(43) Date of publication:
**14.02.2024 Bulletin 2024/07**

(21) Application number: **22784133.5**

(22) Date of filing: **08.04.2022**

(51) International Patent Classification (IPC):
**B01J 8/24** (2006.01)          **B01J 8/00** (2006.01)
**C07C 253/18** (2006.01)        **C07C 255/08** (2006.01)

(52) Cooperative Patent Classification (CPC):
**B01J 8/00; B01J 8/24; C07C 253/18; C07C 255/08**

(86) International application number:
**PCT/CN2022/085766**

(87) International publication number:
**WO 2022/214067 (13.10.2022 Gazette 2022/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.04.2021   CN 202110382764**
**29.01.2022   CN 202210111952**

(71) Applicants:
• **China Petroleum & Chemical Corporation
Beijing 100728 (CN)**

• **Shanghai Research Institute of Petrochemical
Technology SINOPEC
Pudong New Area
Shanghai 201208 (CN)**

(72) Inventors:
• **ZHAO, Le
Shanghai 201208 (CN)**
• **WU, Lianghua
Shanghai 201208 (CN)**

(74) Representative: **karo IP
karo IP Patentanwälte
Kahlhöfer Rößler Kreuels PartG mbB
Postfach 32 01 02
40416 Düsseldorf (DE)**

(54) **COOLING COIL SET, METHOD FOR INCREASING REACTION LOAD, AND METHOD FOR PREPARING UNSATURATED NITRILE**

(57)    The present application relates to a heat removal tube set, a method for increasing reaction load by using the heat removal tube set, and application of the same in the production of unsaturated nitrile. The heat removal tube set comprises at least 10 heat removal tubes, and in at least one and at most 88% of the total of the heat removal tubes of the heat removal tube set, an angle formed between an extended line of the central axis of at least one connecting fitting and an extended line of the central axis of other connecting fitting is greater than 0° and less than 180°. By arranging such a heat removal tube set, the heat removal capability and fluidization efficiency of the fluidized bed reactor is improved, so that the demand for increasing the reaction load can be fully satisfied.

Fig. 7

EP 4 321 243 A1

## Description

## Technical Field

The present application relates to a heat removal tube set particularly suitable for use in a fluidized bed reactor. The present application further relates to a method for increasing the reaction load by using the heat removal tube set and application thereof in the production of unsaturated nitrile.

## Background Art

Acrylonitrile is an important chemical raw material for petrochemical industry. The one-step method for producing acrylonitrile by propylene ammoxidation is commonly adopted in various countries in the world, namely, under the action of a fluidized bed ammoxidation catalyst and under a certain reaction temperature and pressure, the propylene is subjected to ammoxidation to generate acrylonitrile, and at the same time generate byproducts such as acetonitrile, hydrocyanic acid and the like, and deep oxidation products like CO, $CO_2$ are also generated. The reaction is strongly exothermic and is accompanied by the generation of a large amount of heat.

Typical internals of acrylonitrile fluidized bed reactor include a propylene-ammonia distributor, an air distribution plate, a heat removal tube (also known as cooling coil) and a cyclone separator, wherein the heat removal tube and the dipleg of the cyclone separator are disposed in the catalyst bed as vertical members of the fluidized bed. The heat removal tube can remove a large amount of generated reaction heat out of the reaction system in time and maintain the reaction temperature in a stable state, and the cyclone separator captures the catalyst carried by the gas moving upwards and returns the catalyst to the catalyst bed through the dipleg so as to reduce the loss of the catalyst.

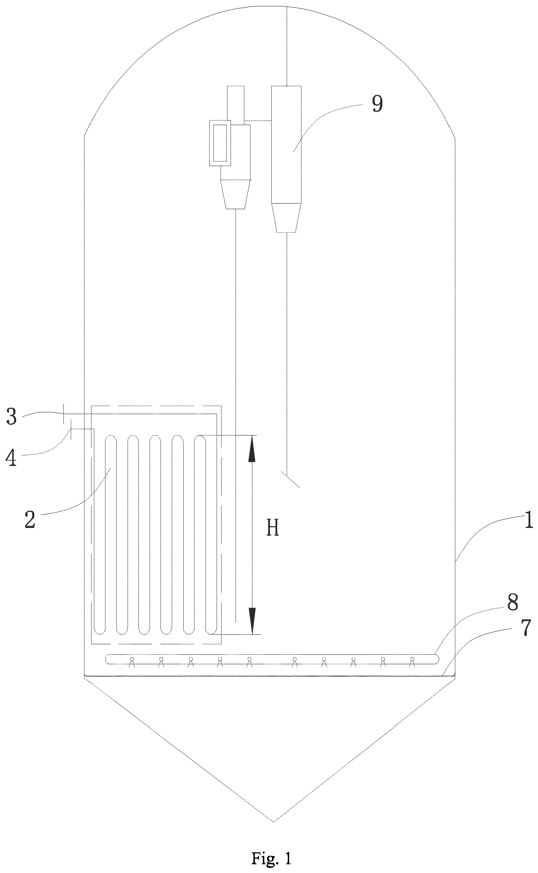

Fig. 1 shows an acrylonitrile fluidized bed reactor, of which the internals mainly include: a distribution plate for oxygen-containing gas, a propylene-ammonia distributor, a heat removal tube and a cyclone separator. In an existing acrylonitrile reactor shown in Fig. 1, 85% or more of the total heat removal tubes are in operation, i.e., those heat removal tubes are filled with a cold heat removal medium relative to the reaction temperature, and the reaction temperature is maintained stable by heat exchange with the heat removal medium.

The improvement of the performance of the ammoxidation catalyst allows the fluidized bed reactor to operate at a greater (e.g., 50% higher) reaction load at the same size, namely, the feeding amount of the raw materials of propylene, ammonia and oxygen-containing gas is increased by 50%, which leads to a 50% increase of the reaction heat released. Although a part of heat removal tubes in existing fluidized bed reactor are in idle state, the additional reaction heat cannot be removed sufficiently, which leads to a runaway of reaction temperature; or alternatively, due to the lack of sufficient heat removal tubes for alternate use, though the reaction temperature can be maintained stable in the initial stage of the operation of the equipment, the molybdenum scale on the surfaces of the heat removal tubes will be increased, the heat conduction efficiency will be reduced, and more heat removal tubes will be required to be in an operation state as the operation period of the equipment increases. Finally, no idle heat removal tubes are available for switching, and the stable control of the reaction temperature cannot be maintained, so that the requirement of long-period stable operation of the equipment cannot be met. On the other hand, since the primary bubbles generated when the gases raw materials leave the distribution plate/distributor become larger, and thus, in the case of existing fluidized bed reactor, the bubbles also become relatively larger throughout the bed, so that the acrylonitrile yield and the propylene conversion are lowered, which is adverse to the economy of the equipment. Existing fluidized bed reactors are limited on heat removal capacity and fluidization efficiency, and thus cannot meet the need for the increase of reaction load.

## Disclosure of the Invention

In the acrylonitrile fluidized bed reactor of the present application, the heat removal tubes are arranged in parallel to each other with a same spacing, or to be vertical to each other with a service passage being kept within the spacing between straight pipes of two adjacent heat removal tubes. Conventional heat removal tubes are characterized in that an upper connecting fitting, a lower connecting fitting and a straight pipe of the heat removal tube are projected on the same straight line on the cross section of the reactor, and the upper connecting fitting and the lower connecting fitting form an angle of 180 degrees; while profiled heat removal tubes are characterized in that at least one upper connecting fitting forms a certain angle with a lower connecting fitting, and the projection of straight pipes in fluid communication on the cross section of the reactor along the flow direction of the main body of the fluid is two closely adjacent straight lines; because profiled heat removal tubes have closer connection compared with conventional heat removal tubes, the heat removal capacity is continuously improved along with the increase of the number of conventional heat removal tubes replaced by profiled heat removal tubes, which is also more beneficial to breaking large bubbles. Meanwhile, enough space is still reserved between heat removal tubes for the maintaining and recondition of the equipment. The present application has been made based on this finding, and has been accomplished by changing the configuration and number of heat removal tubes in a fluidized bed reactor to enhance the heat removal capability and fluidizing efficiency of the fluidized bed reactor. Conventional heat removal tubes and profiled heat removal tubes are configured at a predetermined proportion according to the performance of the ammoxidation catalyst and the reaction load, so that a high-efficiency and long-term stable operation of the equipment can be realized.

Specifically, the present application relates to technical solutions of the following aspects.

1. A heat removal tube set (particularly a heat removal water tube set), characterized in that it is configured to be arranged in a heat removal section of a fluidized bed reactor, said heat removal tube set comprising at least 10 (preferably 10 to 100, more preferably 20 to 80) heat removal tubes, said heat removal tube comprising N (N is equal to or greater than 3, preferably N is 3 to 30, more preferably N is 3 to 20) straight pipes and N-1 connecting fittings for connecting any two adjacent straight pipes thereof in series and providing a fluid communication between them,

where the length of the heat removal section along the central axis of the fluidized bed reactor is set as H (in m), a cross section of the heat removal section (referred to as Cross Section A) is obtained by transecting along a direction perpendicular to the central axis of the fluidized bed reactor at a position within the entire region of the length H of the heat removal section (preferably, within the region from 49% H above to 49% H below the central point of the reaction heat removal section, more preferably, within the region from 45% H above to 38% H below the central point of the reaction heat removal section, more preferably, within the region from 40% H above to 8% H below the central point of the reaction heat removal section),

for at least one (preferably 1, 2 or 3, or at least 20%, at least 50% or at least 65% of the total) of the heat removal tubes (referred to as profiled heat removal tubes) of the heat removal tube set and at most up to 88% (preferably 75% or 70%) of the total of the heat removal tubes of the heat removal tube set, an angle formed between an extended line of the central axis of the projection of at least one (preferably at least 2, 3 or 4, and at most 80%, 90% or 100% of the total) of the connecting fittings of the profiled heat removal tube (preferably excluding the first connecting fitting of the profiled heat removal tube) on the Cross Section A and an extended line of the central axis of the projection of at least one other connecting fitting (preferably the other connecting fitting on the profiled heat removal tube at a position immediately upstream of and in fluid communication with said connecting fitting) on the Cross Section A is greater than 0° and less than 180° (preferably 30°-150°, more preferably 60°-120°, and further preferably about 90°).

2. The heat removal tube set as described in any of the preceding or subsequent aspects, where the area of the Cross Section A is set as S1 (in $m^2$), and where the sum of the outer contour circumferences of the cross sections of all of the straight pipes of the heat removal tube set on the Cross Section A is set as L1 (in m), L1/S1 is 1.0 to 6.0 $m^{-1}$ (preferably 2.4 to 5.6 $m^{-1}$, more preferably 2.9 to 5.3 $m^{-1}$), and/or the area S1 is 20 to 700 $m^2$ (preferably 35 to 350 $m^2$), and/or the L1 is 20 to 4200m, preferably 87.5 to 1225 m.

3. The heat removal tube set as described in any of the preceding or subsequent aspects, where the total number of the straight pipes in the heat removal tube set is set as Nt, the number of straight pipes per unit area of the Cross Section A, i.e. Nt/S1, is 4-16/$m^2$ (preferably 5-14/$m^2$, and more preferably 7-13/$m^2$), and/or the profile of the Cross Section A is circular, elliptical or oval, and preferably circular or substantially circular, and/or the inner profile and the outer contour of the cross section of the straight pipe are circular, elliptical or oval, and preferably circular or substantially circular.

4. The heat removal tube set as described in any of the preceding or subsequent aspects, characterized in that the heat removal tube set is capable of recovering 1-10 MPa saturated steam (preferably 2-8 MPa saturated steam, more preferably 3-5 MPa saturated steam), and/or has a heat removal capacity of 0.5-3.0 t saturated steam per unit cross-sectional area ($m^2$) per hour, preferably 1.0-2.8 t saturated steam per unit cross-sectional area ($m^2$) per hour, more preferably 1.2-2.4 t saturated steam per unit cross-sectional area ($m^2$) per hour, when evaluated as recovering 4.5MPa saturated steam, wherein said unit cross-sectional area refers to the unit area of the Cross Section A.

5. The heat removal tube set as described in any of the preceding or subsequent aspects, wherein for heat removal tube(s) of said heat removal tube set other than said profiled heat removal tube, an angle formed between an extended line of the central axis of the projection of any connecting fitting on said Cross Sectional A and an extended line of the central axis of the projection of another connecting fitting on said heat removal tube at a position immediately upstream or downstream of and in fluid communication with said connecting fitting on said Cross Sectional A is 180°, and/or wherein said heat removal tube comprises a cooling water inlet, and the cooling water inlets of a plurality (preferably 2-8, 2-6 or 2-4) of said heat removal tubes are merged into a cooling water inlet header in said heat removal section, and/or wherein said heat removal tube comprises a cooling water outlet, and the cooling water outlets of a plurality (preferably 2-8, 2-6 or 2-4) of said heat removal tubes are merged into a cooling water outlet header in the heat removal section.

6. The heat removal tube set as described in any of the preceding or subsequent aspects, characterized in that the outer diameters of the straight pipes are respectively 80-180 mm, preferably 90-170 mm, and/or the inner diameters of the straight pipes are respectively 60-150 mm, preferably 70-140 mm, and/or the lengths of the straight pipes are respectively 4-13 m, preferably 5-12.0 m, and/or the spacing between two adjacent straight pipes on each heat removal tube is 100-700 mm, preferably 150-300 mm, and/or the length H of the heat removal section is 4-13 m (preferably 5-12 m).

7. The heat removal tube set as described in any of the preceding or subsequent aspects, characterized in that,

(1) where the full propylene treatment capacity per hour of the fluidized bed reactor is 140-290 kg propylene/$m^2$ of the Cross Section A, excluding the endpoint of 290, L1/S1 is 1.0-2.5 $m^{-1}$ (excluding the endpoint of 2.5), preferably 1.4-2.2 $m^{-1}$, or

(2) where the full propylene treatment capacity per hour of the fluidized bed reactor is 200-370 kg propylene/$m^2$ of the Cross Section A, L1/S1 is 1.8-4.6 $m^{-1}$, preferably 2.0-4.1 $m^{-1}$, or

(3) where the full propylene treatment capacity per hour of the fluidized bed reactor is 290-445 kg propylene/$m^2$ of the Cross Section A, L1/S1 is 2.5-6.0 $m^{-1}$, preferably 2.9-5.3 $m^{-1}$.

8. A fluidized bed reactor, characterized in that it comprises a head, a dilute phase zone, a heat removal section, a pre-reaction section and a cone from top to bottom in sequence, wherein a heat removal tube set as described in any of the preceding or subsequent aspects is arranged in the heat removal section.

9. A method for producing an unsaturated nitrile, comprising the step of subjecting an olefin (such as propylene) to an ammoxidation reaction in a fluidized bed reactor as described in any of the preceding or subsequent aspects to obtain an unsaturated nitrile (such as acrylonitrile).

10. A method for increasing the load of a fluidized bed reactor, wherein the full propylene treatment capacity per hour of the fluidized bed reactor is 140-290 kg propylene/$m^2$ of the Cross Section A (excluding the endpoint of 290) and L1/S1 is 1.0-2.5 $m^{-1}$ (excluding the endpoint of 2.5), the method comprising increasing L1/S1 to 2.5-6.0 $m^{-1}$, preferably to 2.9-5.3 $m^{-1}$, while increasing the full propylene treatment capacity per hour of the fluidized bed reactor to 290-445 kg propylene/$m^2$ of the Cross Section A.

11. A method for producing an unsaturated nitrile, comprising the step of subjecting an olefin (such as propylene) to an ammoxidation reaction in a fluidized bed reactor to obtain an unsaturated nitrile (such as acrylonitrile), wherein the load of the fluidized bed reactor is increased according to the method for increasing the load as described in any of the preceding or subsequent aspects.

12. The method as described in any of the preceding or subsequent aspects, wherein the molar ratio of propylene/ammonia/air (calculated as molecular oxygen) is 1 : 1.1-1.3 : 1.8-2.0, the reaction temperature is 420-440 °C, the reaction pressure (gauge pressure) is 0.03-0.14 MPa, and the weight hourly space velocity of the catalyst is 0.06-0.15 $h^{-1}$.

**Brief Description of the Drawings**

Fig. 1 is a schematic front view of an existing fluidized bed reactor.
Fig. 2 is a schematic top view of an existing reaction heat removal tube set for fluidized bed.
Fig. 3 is a schematic top view of a reaction heat removal tube set for fluidized bed according to the present application.
Figs. 4A-4D are schematic views of the arrangement of the heat removal tubes of the heat removal tube set according to the present application.
Fig. 5 is a diagram of the pressure pulsation intensity.
Figs. 6 and 7 are schematic views of the arrangement of the heat removal tube headers of the present application.

Description of the reference numerals:

1: wall of fluidized bed reactor
2: heat removal tube
3: cooling water inlet of heat removal tube
4: cooling water outlet of heat removal tube
5: lower connecting fitting of heat removal tube
6: upper connecting fitting of heat removal tube
7: distribution plate for oxygen-containing gas
8: propylene-ammonia distributor
9: high-efficiency cyclone separator

**Technical effects**

By using the heat removal tube set and the fluidized bed reactor according to the present application, the production capacity of a target product of the equipment can be improved, the requirement for improving the reaction load can be fully met, and the operation cost of the equipment can be reduced.

In the heat removal tube set according to the present application, a closer arrangement can be realized, namely more straight pipes can be arranged per unit cross section area of the reactor, thereby enhancing the heat removal capability.

By using the heat removal tube set and the fluidized bed reactor according to the present application, the change of the flow pattern in the fluidized bed can be accelerated, and the mass transfer efficiency can be improved.

By using the heat removal tube set and the fluidized bed reactor according to the present application, the growth of bubbles can be effectively inhibited, so that the conversion rate of feed gas can be improved and the yield of target reaction product can be increased.

By using the heat removal tube set and the fluidized bed reactor according to the present application, the back mixing degree of gas and solid phases can be reduced, and the generation of deep oxidation products can be reduced.

By using the heat removal tube set and the fluidized bed reactor according to the present application, the heat transfer efficiency can be improved, and the operation period of the equipment can be prolonged.

**Detailed Description of the Invention**

The present application will be illustrated in detail hereinbelow with reference to embodiments thereof, but it should be noted that the scope of the present application is not limited by those embodiments, but is defined by the appended claims.

All publications, patent applications, patents, and other references cited herein are incorporated by reference in their entirety. Unless defined otherwise, all technical and scientific terms used herein have the same meaning as commonly understood by those skilled in the art. In case of conflict, the contents described herein, including definitions, should prevail.

Where a material, method, component, device, or equipment is described herein as "known to those skilled in the art", "conventionally known in the art" or the like, it is to be understood that said material, method, component, device, or equipment cover not only those conventionally used in the art at the time of filing the present application, but also those not commonly used at present but will become commonly known in the art to be suitable for a similar purpose.

In the context of the present application, the term "substantially" means that a deviation acceptable or considered reasonable by those skilled in the art may be present, such as a deviation within $\pm10\%$, within $\pm5\%$, within $\pm1\%$, within $\pm0.5\%$ or within $\pm0.1\%$.

In the context of the present application, unless specifically stated otherwise, all percentages, parts, ratios, etc. are expressed by weight and all pressures given are gauge pressures.

In the context of the present application, any two or more embodiments of the present application may be arbitrarily combined, and the resulting technical solution forms a part of the initial disclosure of the present application and falls within the scope of the present application.

According to an embodiment, the present application relates to a heat removal tube set, particularly a heat removal water tube set. According to the present application, a "heat removal tube set" and "heat removal tube" may be used to remove excess heat from a reactor in which an exothermic reaction (or some exothermic stages of the reaction) is conducted, to maintain the reaction within a certain temperature range. Examples of the reactor include a fluidized bed reactor, and more particularly, a fluidized bed reactor for producing acrylonitrile.

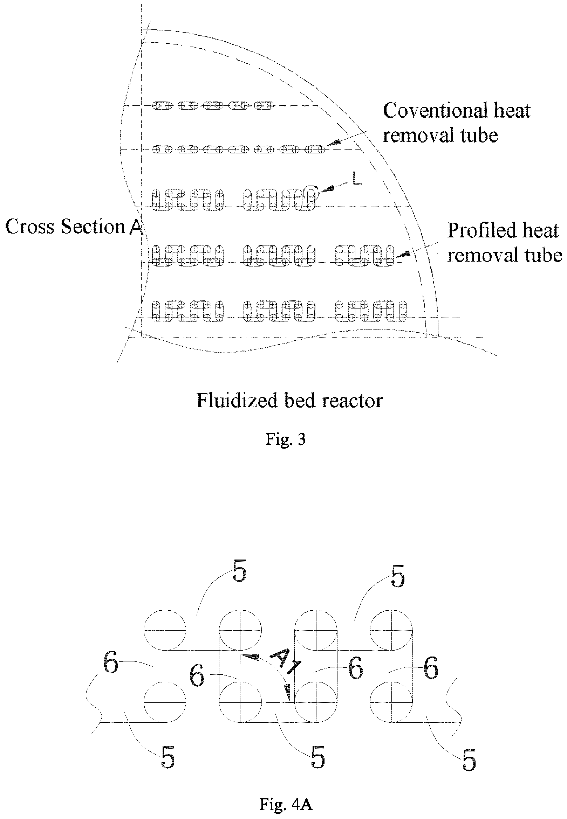

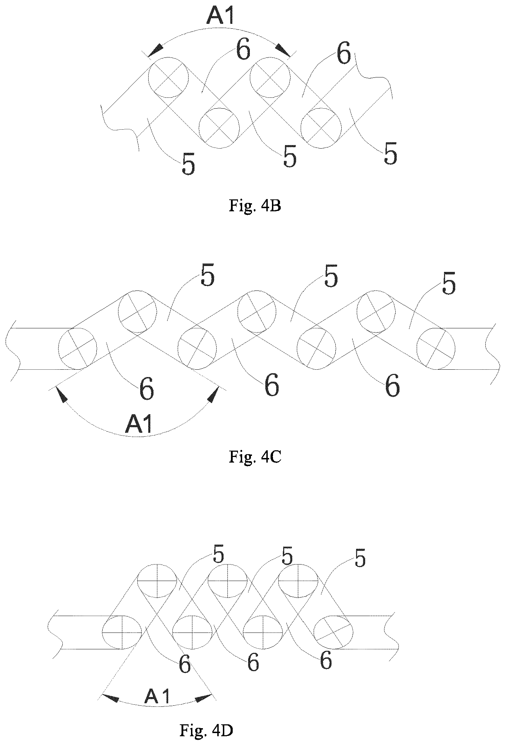

According to an embodiment of the present application, the heat removal tube set comprises at least 10 (preferably 10 to 100, more preferably 20 to 80) heat removal tubes. Typically, the heat removal tubes comprise a cooling water inlet, straight pipes and a cooling water outlet, and connecting fittings for connecting these pipes in a fluid communication manner. Preferably, the heat removal tube comprises N (N is equal to or greater than 3, preferably, N is 3 to 30, and more preferably, N is 3 to 20) straight pipes and N-1 connecting fittings for connecting any two adjacent straight pipes thereof in series and providing a fluid communication between them. As shown in Fig. 3, each heat removal tube 2 comprises: cooling water inlet 3, cooling water outlet 4, at least 3 adjacent straight pipes and connecting fittings for connecting any two adjacent straight pipes thereof in series and providing a fluid communication between them. As shown in Fig. 4, where a connecting fitting for connecting any two straight pipes is positioned below the straight pipes (hereinafter, sometimes simply referred to as "lower connecting fitting 5"), another connecting fitting adjacent thereto is positioned above the straight pipes (hereinafter, sometimes simply referred to as "lower connecting fitting 6").

In the context of the present application, the straight pipe closest to the cooling water inlet of a heat removal tube is referred to as a first connecting fitting. In addition, in a heat removal tube, each straight pipe shows an upstream-downstream positional relationship along the flow direction of the cooling water. In the present application, a position that is adjacent and upstream is referred to as an immediately upstream position, and a position that is adjacent and downstream is referred to as an immediately downstream position.

According to an embodiment of the present application, the heat removal tube set is configured to be arranged in a heat removal section of a fluidized bed reactor. Obviously, the heat removal tube is also configured to be arranged in the heat removal section of the fluidized bed reactor. Specifically, the straight pipes of the heat removal tubes are basically positioned in the dense-phase area of the fluidized bed reactor and are used for timely removing reaction heat out of the system and maintaining a stable operation of the system. For this reason, in the context of the present specification, the "heat removal section" refers to the region of the fluidized bed reactor in which the heat removal tubes are disposed, more particularly the region of the fluidized bed reactor in which the straight pipes of the heat removal tubes are disposed, more particularly the region in the dense phase region of the fluidized bed reactor in which the straight pipes of the heat removal tubes are disposed.

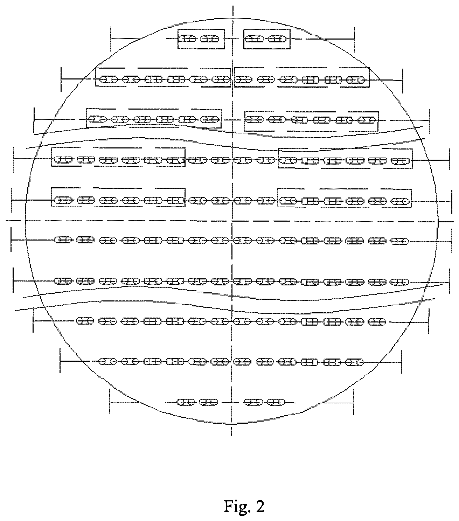

In prior arts, the heat removal tube sets in the heat removal section are typically arranged in the manner shown in Fig. 2, i.e., the heat removal tubes are arranged in a straight line. On the other hand, as shown in Fig. 1, other internal components such as dipleg of cyclone 9 are also included in the heat removal section of the fluidized bed reactor. For this reason, in order to further improve the fluidization conditions and the high capacity requirements of the equipment, it is possible that existing heat removal tube sets are not sufficient to enable a normal functioning of the equipment.

According to an embodiment of the present application, where the length of the heat removal section along the central axis of the fluidized bed reactor is set as H (in m), a cross section of the heat removal section (referred to as Cross Section A) is obtained by transecting along a direction perpendicular to the central axis of the fluidized bed reactor at a position within the entire region of the length H of the heat removal section. Here, the cross section of the heat removal section refers to the cross section of the inner contour of the fluidized bed reactor at the heat removal section. As the region, it is preferably within the region from 49% H above to 49% H below the center point of the reaction heat removal section, more preferably within the region from 45% H above to 38% H below the center point of the reaction heat removal section, and still more preferably within the region from 40% H above to 8% H below the center point of the reaction heat removal section.

According to an embodiment of the present application, the heat removal tube set comprises a profiled heat removal tube. Here, the number of the profiled heat removal tubes in the heat removal tube set is at least 1, preferably 1, 2 or 3, or at least 20%, at least 50% or at least 65% of the total of the heat removal tubes of the heat removal tube set. In addition, from the viewpoint of optimally achieving the technical effects of the present application, the number of the profiled heat removal tubes is up to 88%, preferably 75% or 70%, of the total of the heat removal tubes in the heat removal tube set. Theoretically, in order to maintain the consistency of the radial reaction temperature, it is desirable that heat removal straight pipes in work are uniformly distributed on the cross section of the reactor. However, in fact, the reaction heat is changed along with the change of the feed amount of the feed gas, and for the heat removal straight pipes fixed in the bed layer of the reactor, heat removal straight pipes in work cannot be completely uniformly distributed, but it is preferably those heat removal straight pipes are uniformly distributed as far as possible, so that the radial reaction temperature difference is as small as possible, for example, within 3 °C. The heat emitted by chemical reaction can be estimated, heat removal straight pipes needed can also be calculated in advance. Since the heat removal capacity of the region where the profiled heat removal tube is disposed is greatly increased, the distance between adjacent profiled heat removal tubes in work is increased, and although the heat transfer efficiency of the fluidized bed is high, there is a risk that reaction heat cannot be removed in time from the area between two profiled heat removal tubes. If all (100%) of the heat removal tubes in the heat removal tube set are profiled heat removal tubes, the desired technical effect of the present application cannot be effectively achieved.

According to an embodiment of the present application, the profiled heat removal tube refers to a heat removal tube in which an angle (i.e. angle A) formed between an extended line of the central axis of the projection of at least one connecting fitting (referred to as special connecting fitting) on the Cross Section A and an extended line of the central axis of the projection of at least one other connecting fitting on the Cross Section A is greater than 0° and less than 180°. The angle A is preferably 30°-150°, more preferably 60°-120°. From the perspective of realizing an optimal technical effect of the present application, the angle A is most preferably about 90°, so that a close arrangement of the heat removal straight pipes can be realized. In addition, the number of the special connecting fittings in a profiled heat removal tube is preferably at least 2, 3 or 4, and is at most 80%, 90% or 100% of the total of the connecting fittings in the profiled heat removal tube. From the perspective of realizing an optimal technical effect, the number of the special connecting fittings is preferably 100% of the total of the connecting fittings in the profiled heat removal tubes, so that the heat removal tubes are arranged in parallel to each other with the same spacing, while maintaining enough service passages in the equipment.

According to an embodiment of the present application, as said at least one other connecting fitting, it is preferable other connecting fitting on the profiled heat removal tube at a position immediately upstream of and in fluid communication with the connecting fitting. In addition, the first connecting fitting of the profiled heat removal tube is normally excluded, in view of the situation that there is no position immediately upstream of the first connecting fitting. Nevertheless, the angle (i.e. Angle A1) formed between an extended line of the central axis of the projection of the first connecting fitting on the Cross Section A and an extended line of the central axis of the projection of the cooling water inlet on the Cross Section A may be any value, for example 30°-180°, 60°-180°, 90° or 180°. For the same reason as described above, the Angle A1 is also preferably 90°.

Specifically, in the projection on the cross section, the straight pipes and the connecting fittings of the same heat removal tube 2 may be arranged in the manner shown in Fig. 4A to 4D, for example.

According to an embodiment of the present application, the straight pipes of heat removal tube 2 in the heat removal tube set have a substantially circular cross-sectional outer contour with an outer contour circumference of 3.14×D. Here, D is the diameter of the outer contour of the straight pipe. Therefore, in the cross section of the heat removal section shown in Fig. 3, the sum L1 of the outer contour circumference of the cross sections of all of the straight pipes of the heat removal tube set is the sum of the outer contour circumferences of all of the straight pipes in the cross section of the heat removal section.

According to an embodiment of the present application, where the area of the Cross Section A is set as S1 (in $m^2$) and the sum of the outer contour circumferences of the cross sections of all of the straight pipes of the heat removal tube set on the Cross Section A is set as L1 (in m), L1/S1 is 1.0 to 6.0 $m^{-1}$ (preferably 2.4 to 5.6 $m^{-1}$, more preferably 2.9 to 5.3 $m^{-1}$). Here, L1/S1 represents the distribution density of all heat removal tubes (or straight pipes) on the Cross Section A, of which there is an optimum range for the achievement of the technical effect of the present application. When the distribution density of L1/S1 is lower than 1.0, the operation reaction load of the equipment is low, so that the production cost is high and the economy is poor for enterprises, and when the distribution density of L1/S1 is higher than 6.0, the equipment can be operated at high reaction load, so that the requirement for removing more reaction heat can be met, the number of profiled heat removal tubes in the reactor can be increased, and the stability of the reaction temperature may be harmed in the operation process, or the space for maintenance may be compressed.

According to an embodiment, the present application relates to a heat removal tube set, characterized in that the heat removal tube set is arranged in a heat removal section of a fluidized bed reactor, the heat removal tube set comprises at least one (preferably 10 to 100, and more preferably 20 to 80) heat removal tubes, the heat removal tubes comprise N (N is more than or equal to 3, preferably N is 3 to 30, and more preferably N is 3 to 20) straight pipes and N-1 connecting fittings for connecting any two adjacent straight pipes thereof in series and providing a fluid communication between them, wherein where the length of the heat removal section along the central axis of the fluidized bed reactor is set as H (in m), and the area of a cross section of the heat removal section obtained by transecting along a direction perpendicular to the central axis of the fluidized bed reactor at a position within the entire region of the length H of the heat removal section (preferably, within the region from 49% H above to 49% H below the central point of the reaction heat removal section, more preferably, within the region from 45% H above to 38% H below the central point of the reaction heat removal section, more preferably, within the region from 40% H above to 8% H below the central point of the reaction heat removal section) is set as S1 (in $m^2$), in the heat removal tube, an angle formed between an extended line of the central axis of the projection of at least one connecting fitting on the cross section and an extended line of the central axis of the projection of at least one other connecting fitting on the cross section is greater than 0° and less than 180°,

(preferably 30°-150°, more preferably 60°-120°, more preferably 90°), and in the cross section, the sum of the outer contour circumferences of the cross sections of all of the straight pipes of the heat removal tube set is set as L1 (in m), then L1/S1 is 1.0-6.0 $m^{-1}$ (preferably 2.0-4.0 $m^{-1}$, more preferably 2.5-3.5 $m^{-1}$).

According to an embodiment of the present application, said area S1 is between 20 and 700 $m^2$ (preferably between 35 and 350 $m^2$).

According to an embodiment of the present application, the L1 is 20 to 4200 m, preferably 87.5 to 1225 m.

According to an embodiment of the present application, where the total number of the straight pipes in the heat removal tube set is set as Nt, the number of straight pipes per unit area of the Cross Section A, i.e. Nt/S1, is 4-16/$m^2$ (preferably 5-14/$m^2$, and more preferably 7-13/$m^2$). Here, Nt/S1 also represents the distribution density of heat removal tubes (or straight pipes) on the Cross Section A, of which there is an optimum range for the achievement of the technical effect of the present application. When the distribution density of Nt/S1 is less than 4, it is not beneficial to breaking bubbles for the fluidized bed , so that more feed gas is carried out of the reactor along with the gas without participating in the reaction, and the reaction result is influenced; and when the distribution density of Nt/S1 is more than 16, a high-load operation of the reaction can be met, but there is a risk of unstable reaction temperature during the operation process and compressing of the space for maintenance due to the increase of profiled heat removal tubes.

According to an embodiment of the present application, the profile of the Cross Section A is circular, elliptical or oval, preferably circular or substantially circular.

According to an embodiment of the present application, the inner and outer contours of the cross section of the straight pipe are circular, elliptical or oval, preferably circular or substantially circular.

According to an embodiment of the present application, the heat removal tube set is capable of recovering from 1 to 10 MPa of saturated steam, preferably from 2 to 8 MPa of saturated steam, more preferably from 3 to 5MPa of saturated steam. Particularly, the heat removal capacity of the heat removal tube set, when evaluated as recovering 4.5MPa saturated steam, is 0.5 to 3.0 t saturated steam per unit cross-sectional area ($m^2$) per hour, preferably 1.0 to 2.8 t saturated steam per unit cross-sectional area ($m^2$) per hour, more preferably 1.2 to 2.4 t saturated steam per unit cross-sectional area ($m^2$) per hour, where the unit cross-sectional area refers to the unit area of the Cross Section A. Compared with the prior art, the heat removal tube set can recover more saturated steam, thereby showing stronger heat removal capacity and meeting the requirement of high reaction load.

According to an embodiment of the present application, the heat removal tube set further comprises a conventional heat removal tube, and the conventional heat removal tubes and the profiled heat removal tubes together form the heat removal tube set. Here, in a conventional heat removal tube, an angle formed between an extended line of the central axis of the projection of any one of the connecting fittings on the Cross Sectional A and an extended line of the central axis of the projection of other connecting fitting on the heat removal tube at a position immediately upstream or downstream of and in fluid communication with the connecting fitting on the Cross Sectional A is 180°. In short, as the conventional heat removal tube, all of the straight pipes are disposed in the same plane.

In the context of the present application, a heat removal tube encompasses both a profiled heat removal tube and a conventional heat removal tube, unless explicitly indicated otherwise.

According to an embodiment of the present application, a plurality (preferably 2-8, 2-6 or 2-4) of the cooling water inlets of the heat removal tubes are merged into a cooling water inlet header in the heat removal section. In other words, the plurality of heat removal tubes share one cooling water inlet. According to the present application, the cooling water inlet header is in fluid communication with an external cooling water supply source through the wall of the fluidized bed reactor, whereby cooling water is supplied to each heat removal tube through the cooling water inlet header. The heat removal tube here (referred to as a heat removal branch pipe) may be a conventional heat removal tube, a profiled heat removal tube, or any combination thereof, and is not particularly limited.

According to an embodiment of the present application, a plurality (preferably 2-8, 2-6 or 2-4) of the cooling water outlets of the heat removal tubes are merged into a cooling water outlet header in the heat removal section. In other words, the plurality of heat removal tubes share one cooling water outlet. According to the present application, the cooling water outlet header is in fluid communication with an external cooling water receiving means through the wall of the fluidized bed reactor, whereby cooling water (typically further comprising steam) after heat removal is delivered from the heat removal tubes to the external environment through the cooling water outlet header. The heat removal tube here (referred to as a heat removal branch pipe) may be a conventional heat removal tube, a profiled heat removal tube, or any combination thereof, and is not particularly limited.

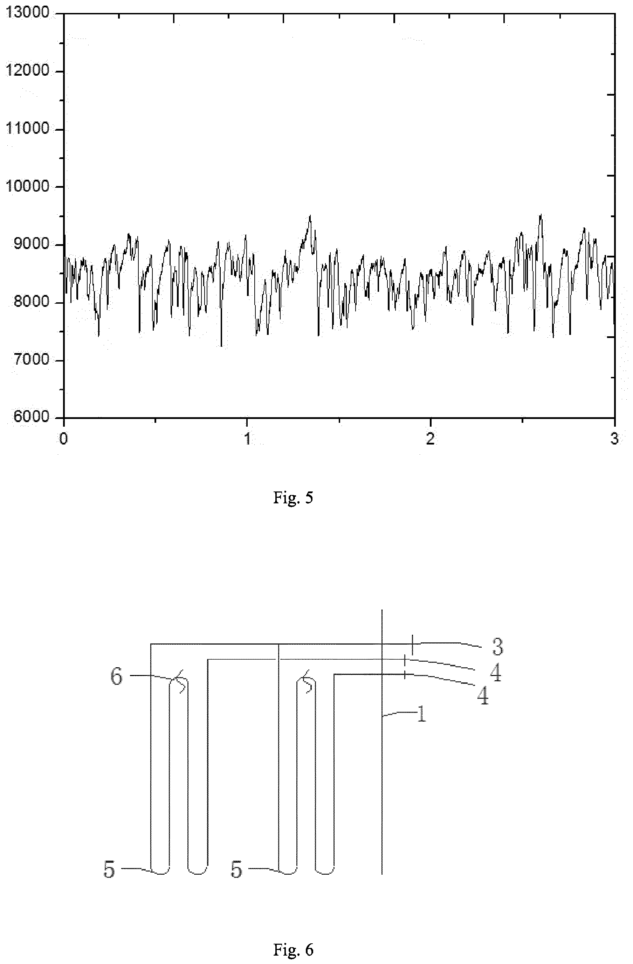

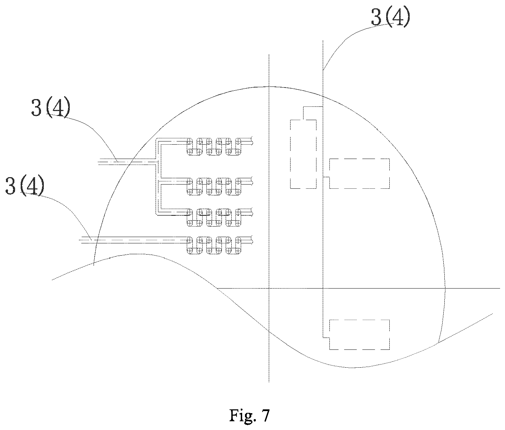

Figs. 6 and 7 are schematic views of the arrangement of the heat removal tube headers of the present application. As can be seen from the figures, the cooling water inlets/outlets of the plurality of heat removal tubes are merged into a header.

According to an embodiment of the present application, a ratio of a cross-sectional area of the header (such as the cooling water inlet header or the cooling water outlet header) to a sum of cross-sectional areas of the plurality of heat removal branch pipes (generally, calculated based on the cooling water inlets or the cooling water outlets of the plurality of heat removal branch pipes) corresponding thereto is 0.5 to 1, preferably 0.55 to 0.95, and more preferably 0.6 to 0.9. The proportion of the heat removal branch pipes in the total of the heat removal tubes is typically 66% or less, preferably 50% or less, and more preferably 33% or less.

According to an embodiment of the present application, the outer diameters of the straight pipes are each independently 80-180 mm, preferably 90-170 mm.

According to an embodiment of the present application, the inner diameters of the straight pipes are each independently 60-150 mm, preferably 70-140 mm.

According to an embodiment of the present application, the lengths of the straight pipes are each independently 4 to 13 m, preferably 5 to 12.0 m.

According to an embodiment of the present application, the distance between two adjacent straight pipes on each heat removal tube is 100-700 mm, preferably 150-300 mm.

According to an embodiment of the present application, the length H of the heat removal section is 4-13m (preferably 5-12 m).

According to an embodiment of the present application, where the full propylene treatment capacity per hour of the fluidized bed reactor is 140-290 kg propylene/$m^2$ of the Cross Section A (excluding the endpoint of 290), $L1/S1$ is 1.0-2.5 $m^{-1}$ (excluding the endpoint of 2.5), preferably 1.4-2.2 $m^{-1}$. This represents the operating condition of the fluidized bed reactor at a lower operation load.

According to an embodiment of the present application, where the full propylene treatment capacity per hour of the fluidized bed reactor is 200-370 kg propylene/$m^2$ of the Cross Section A, $L1/S1$ is 1.8-4.6 $m^{-1}$, preferably 2.0-4.1 $m^{-1}$. This represents the operating state of the fluidized bed reactor at a middle operation load.

According to an embodiment of the present application, where the full propylene treatment capacity per hour of the fluidized bed reactor is 290-445 kg propylene/$m^2$ of the Cross Section A, $L1/S1$ is 2.5-6.0 $m^{-1}$, preferably 2.9-5.3 $m^{-1}$. This represents the operating conditions of the fluidized bed reactor at a higher operation load and is the most preferred operating conditions of the present application.

According to an embodiment, the present application also relates to a fluidized bed reactor. The reactor sequentially comprises a head, a dilute phase zone, a heat removal section, a pre-reaction section and a cone from top to bottom, wherein a heat removal tube set as described in any one of the previous embodiments in the present application is arranged in the heat removal section.

According to an embodiment, the present application also relates to a method for producing an unsaturated nitrile, particularly a method for producing acrylonitrile. The method comprises the step of subjecting an olefin (such as propylene) to an ammoxidation reaction in a fluidized bed reactor as described in any one of the preceding embodiments to obtain an unsaturated nitrile (such as acrylonitrile).

According to an embodiment, the present application also relates to a method for increasing the reaction load of a fluidized bed reactor. Here, as the initial reaction load of the conveying fluidized bed reactor, the full propylene treatment capacity per hour of the conveying fluidized bed reactor is 140-290 kg propylene/m$^2$ of the Cross Section A (excluding the endpoint of 290), the initial value of L1/S1 of the fluidized bed reactor is 1.0-2.5 m$^{-1}$ (excluding the endpoint of 2.5). In order to achieve an increased reaction load, the method comprises increasing the full propylene treatment capacity of the fluidized bed reactor from the initial reaction load to 290-445 kg propylene/m$^2$ of the Cross Section A per hour, and in order to accommodate this increased reaction load to ensure that the reaction can be smoothly conducted, it is necessary to increase the L1/S1 from the initial value to 2.5-6.0 m$^{-1}$, preferably to 2.9-5.3 m$^{-1}$.

According to an embodiment, the present application also relates to a method for producing an unsaturated nitrile, comprising the step of subjecting an olefin (such as propylene) to an ammoxidation reaction in a fluidized-bed reactor to obtain an unsaturated nitrile (such as acrylonitrile), wherein the load of the fluidized-bed reactor is increased according to the method for increasing the load as described in any of the preceding embodiments described in the present specification.

According to an embodiment of the present application, the ammoxidation reaction may be performed in any manner and by any method conventionally known in the art, and such information is known to those skilled in the art and will not be described herein in detail. Nevertheless, as the conditions for the ammoxidation reaction, specific examples thereof include those in which the molar ratio of propylene/ammonia/air (calculated as molecular oxygen) is 1: 1.1-1.3: 1.8-2.0, the reaction temperature is 420-440 °C, the reaction pressure (gauge pressure) is 0.03-0.14 MPa, and the weight hourly space velocity of the catalyst is 0.06-0.15 h$^{-1}$.

**Examples**

The present application will be further illustrated in detail with reference to the following examples and comparative examples, but the present application is not limited to those examples.

In the following examples and comparative examples, the acrylonitrile yield and the propylene conversion can be calculated according to the following equations:

Yield of acrylonitrile:

$$AN\% = C_{AN}/\Sigma C * 100$$

Conversion of propylene:

$$Cc_3\% = (1 - Cc_{3out}/Cc_{3in}) * 100$$

wherein:

$C_{AN}$: molar amount (mol) of carbon contained in AN in the gas at the outlet of the reactor

$\Sigma C$: total molar amount (mol) of carbon in the gas at the outlet of the reactor

$Cc_{3out}$: molar amount (mol) of carbon contained in $C_3$ in the gas at the outlet of the reactor

$Cc_{3in}$: molar amount (mol) of carbon contained in $C_3$ in the gas at the inlet of the reactor.

It is known in the art that the fluidization quality can be evaluated as good if the pressure pulsation intensity of the fluidized bed exhibits a "high frequency low amplitude" characteristic as shown in Fig. 5. In all of the following examples, the pressure pulsation intensity of the fluidized bed was similar to that of Fig. 5.

In the following examples and comparative examples, heat removal tubes that are not specifically arranged are conventional heat removal tubes.

Example 1

The fluidized bed reactor had a diameter of 9 meters, 180 tons of acrylonitrile catalysts of SANC series of Sinopec Shanghai Research Institute of Petrochemical Technology Co., Ltd. were filled therein, 480 straight pipes with the same height were arranged in the reactor, which were divided into 44 heat removal tubes, 12 heat removal tubes were arranged in the manner as shown in Fig. 4A, the number of the straight pipes per unit area of the cross section of the heat removal section at the central point thereof was 7.6/m$^2$, the outer diameter of the heat removal tubes was 89 mm, and L1/S1 was 2.1/m.

The feed rate of propylene was 7700 NM$^3$/h, the full propylene treatment capacity at this time was 227 kg propylene/h/m$^2$ of the Cross Section A, the reaction temperature was 430 °C, the reaction pressure was 0.04MPa, the propylene: ammonia: air was 1: 1.2: 9.6, the reaction could be stably operated for a long time (such as 10000 h), and the heat quantity removed per hour was 1.24t of 4.5 MPa saturated steam/m$^2$.

Example 2

The fluidized bed reactor had a diameter of 9 meters, 180 tons of acrylonitrile catalysts of SANC series of Sinopec Shanghai Research Institute of Petrochemical Technology Co., Ltd. were filled therein, 572 straight pipes with the same height were arranged in the reactor, which were divided into 56 heat removal tubes, 36 heat removal tubes were arranged in the manner shown in Fig. 4A, the number of the straight pipes per unit area of the cross section of the heat removal section at the central point thereof was 9.0/m$^2$, each group was formed by connecting 6, 10 and 12 heat removal tube straight pipes in series through straight pipe connecting fittings, the outer diameter of each heat removal tube was 140 mm, and L1/S1 was 4.0.

The feed rate of propylene was 11800 NM$^3$/h, the full propylene treatment capacity at this time was 349 kg propylene/h/m$^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 1.98t of 4.5 MPa steam/m$^2$.

Example 3

The fluidized bed reactor had a diameter of 9 meters, 180 tons of acrylonitrile catalysts of SANC series of Sinopec Shanghai Research Institute of Petrochemical Technology Co., Ltd. were filled therein, 732 straight pipes with the same height were arranged in the reactor, which were divided into 70 heat removal tubes, 42 heat removal tubes were arranged in the manner shown in Fig. 4A, the number of the straight pipes per unit area of the cross section of the heat removal section at the central point thereof was 11.5/m$^2$, each group was formed by connecting 6, 10 and 12 heat removal tube straight pipes in series through straight pipe connecting fittings, the outer diameter of each heat removal tube was 114 mm, and L1/S1 was 4.1.

The feed rate of propylene was 11800 NM$^3$/h, the full propylene treatment capacity at this time was 349kg propylene/h/m$^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 2.01t of 4.5 MPa steam/m$^2$.

Example 4

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 58 heat removal tubes, 36 heat removal tubes were arranged in the manner shown in Fig. 4B, the number of the straight pipes per unit area of the cross section of the heat removal section at the central point thereof was 9.18/m$^2$, each group was formed by connecting 6, 10 and 12 heat removal tube straight pipes in series through straight pipe connecting fittings, the outer diameter of each heat removal tube was 114 mm, and L1/S1 was 3.3.

The feed rate of propylene was 11800 NM$^3$/h, the full propylene treatment capacity at this time was 349kg propylene/h/m$^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 1.98t of 4.5 MPa steam/m$^2$.

Example 5

The fluidized bed reactor had a diameter of 9 meters, 180 tons of acrylonitrile catalysts of SANC series of Sinopec Shanghai Research Institute of Petrochemical Technology Co., Ltd. were filled therein, 732 straight pipes with the same height were arranged in the reactor, which were divided into 72 heat removal tubes, 52 heat removal tubes were arranged in the manner shown in Fig. 4A, the number of the straight pipes per unit area of the cross section of the heat removal section at the central point thereof was 11.5/m$^2$, each group was formed by connecting 6, 10 and 12 heat removal tube straight pipes in series through straight pipe connecting fittings, the outer diameter of each heat removal tube was 140 mm, and L1/S1 was 5.1.

The feed rate of propylene was 14400 NM$^3$/h, the full propylene treatment capacity at this time was 425kg propylene/h/m$^2$ of the Cross Section A, the reaction temperature was 430 °C, the reaction pressure was 0.04MPa, the propylene: ammonia: air was 1: 1.2: 9.6, the reaction could be stably operated for a long time (such as 10000 h), and the heat quantity removed per hour was 2.40t of 4.5 MPa steam/m$^2$.

Example 6

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes, 32 heat removal tubes were arranged in the manner shown in Fig. 4C, the number of the straight pipes per unit area of the cross section of the heat removal section at the central point thereof was 9.18/m$^2$, each group was formed by connecting 6, 10 and 12 heat removal tube straight pipes in series through straight pipe connecting fittings, the outer diameter of each heat removal tube was 140mm, and L1/S1 was 4.04/m.

The feed rate of propylene was 11800 NM$^3$/h, the full propylene treatment capacity at this time was 349kg propylene/h/m$^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 1.93t of 4.5 MPa steam/m$^2$.

Example 7

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes, 32 heat removal tubes were arranged in the manner shown in Fig. 4D, the number of the straight pipes per unit area of the cross section of the heat removal section at the central point thereof was $9.18/m^2$, each group was formed by connecting 6, 10 and 12 heat removal tube straight pipes in series through straight pipe connecting fittings, the outer diameter of each heat removal tube was 140mm, and L1/S1 was 4.04/m.

The feed rate of propylene was 11800 $NM^3$/h, the full propylene treatment capacity at this time was 349kg propylene/h/$m^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 1.94t of 4.5 MPa steam/$m^2$.

Example 8

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes, 48 heat removal tubes were arranged in the manner shown in Fig. 4A, the number of the straight pipes per unit area of the cross section of the heat removal section at the central point thereof was $9.18/m^2$, each group was formed by connecting 6, 10 and 12 heat removal tube straight pipes in series through straight pipe connecting fittings, the outer diameter of each heat removal tube was 140mm, and L1/S1 was 4.04.

The feed rate of propylene was 11800 $NM^3$/h, the full propylene treatment capacity at this time was 349kg propylene/h/$m^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 1.98t of 4.5 MPa steam/$m^2$.

Comparative Example 1

The fluidized bed reactor had a diameter of 9 meters, 180 tons of acrylonitrile catalysts of SANC series of Sinopec Shanghai Research Institute of Petrochemical Technology Co., Ltd. were filled therein, 380 straight pipes with the same height were arranged in the reactor, which were divided into 36 groups, and arranged in the manner of the heat removal tube set as shown in Fig. 2, the number of the straight pipes per unit area of the cross section of the heat removal section at the central point thereof was $6.0/m^2$, the outer diameter of each heat removal tube was 89mm, and L1/S1 was 1.67/m.

The feed rate of propylene was 11800 $NM^3$/h, the full propylene treatment capacity at this time was 349kg propylene/h/$m^2$ of the Cross Section A, the reaction temperature was 430 °C, the reaction pressure was 0.04MPa, and the propylene: ammonia: air was 1: 1.2: 9.6, during the operation process of the equipment, the reaction temperature could not be stably controlled using the heat removal tube set, and a long-term stable operation of the equipment could not be achieved due to the insufficient quantity of the heat removal tubes.

Comparative Example 2

The fluidized bed reactor had a diameter of 9 meters, 180 tons of acrylonitrile catalysts of SANC series of Sinopec Shanghai Research Institute of Petrochemical Technology Co., Ltd. were filled therein, 584 straight pipes with the same height were arranged in the reactor, which were divided into 56 heat removal tubes, and arranged in the manner of the heat removal tube set as shown in Fig. 2, the number of the straight pipes per unit area of the cross section of the heat removal section at the central point thereof was $9.18/m^2$, each group was formed by connecting 6, 10 and 12 heat removal tube straight pipes in series through straight pipe connecting fittings, the outer diameter of each heat removal tube was 140mm, and L1/S1 was 4.04/m.

The feed rate of propylene was 11800 $NM^3$/h, the full propylene treatment capacity at this time was 349kg propylene/h/$m^2$ of the Cross Section A, the reaction temperature was 430 °C, the reaction pressure was 0.04MPa, and the propylene: ammonia: air was 1: 1.2: 9.6, and the heat quantity removed was 1.98t steam/$m^2$.

Although a normal operation of the equipment could be achieved, the maintenance and recondition of the internal components could not be satisfied when the equipment was stopped.

Comparative Example 3

The fluidized bed reactor had a diameter of 9 meters, 180 tons of acrylonitrile catalysts of SANC series of Sinopec Shanghai Research Institute of Petrochemical Technology Co., Ltd. were filled therein, 532 straight pipes with the same height were arranged in the reactor, which were divided into 46 heat removal tubes, all of the heat removal tubes were arranged in the manner shown in Fig. 4A, the number of the straight pipes per unit area of the cross section of the heat removal section at the central point thereof was $8.37/m^2$, each group was formed by connecting 6, 10 and 12 heat removal tube straight pipes in series through straight pipe connecting fittings, the outer diameter of each heat removal tube was 89 mm, and L1/S1 was 2.34.

The feed rate of propylene was 11800 NM$^3$/h, the full propylene treatment capacity at this time was 349kg propylene/h/m$^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 1.88t of 4.5 MPa steam/m$^2$. As compared to Example 2, the fluidization quality was inferior, resulting in a decrease in propylene conversion and a decrease in the yield of the reaction product, and therefore a decrease in the exothermic heat of reaction.

Example 8

The fluidized bed reactor had a diameter of 9 meters, 180 tons of acrylonitrile catalysts of SANC series of Sinopec Shanghai Research Institute of Petrochemical Technology Co., Ltd. were filled therein, 732 straight pipes with the same height were arranged in the reactor, which were divided into 70 heat removal tubes, 4 groups were formed by connecting 3 heat removal tube sets in parallel, the outer diameter of an inlet header of each heat removal tube was 140 mm, the outer diameter of an outlet header of each heat removal tube was 150 mm, the number of the straight pipes per unit area of the cross section of each heat removal section at the central point thereof was 11.5/m$^2$, each heat removal tube was formed by connecting 6, 10 and 12 heat removal tube straight pipes in series through straight pipe connecting fittings, the outer diameter of each heat removal tube was 89 mm, L1/S1 was 3.2, the ratio of the cross sectional area of the inlet header to the sum of the cross sectional areas of the heat removal branch pipes was 0.82, and the ratio of the cross sectional area of the outlet header to the sum of the cross sectional areas of the heat removal branch pipes was 0.95.

The feed rate of propylene was 11800 NM$^3$/h, the full propylene treatment capacity at this time was 349kg propylene/h/m$^2$ of the Cross Section A, the reaction temperature was 430 °C, the reaction pressure was 0.04MPa, and the propylene: ammonia: air was 1: 1.2: 9.6, the reaction could be stably operated for a long time (such as 10000 h), and the heat quantity removed per hour was 1.98t of 4.5 MPa steam/m$^2$.

Example 9

The fluidized bed reactor had a diameter of 9 meters, 180 tons of acrylonitrile catalysts of SANC series of Sinopec Shanghai Research Institute of Petrochemical Technology Co., Ltd. were filled therein, 732 straight pipes with the same height were arranged in the reactor, which were divided into 70 heat removal tubes, 10 groups were formed by connecting 2 heat removal tube sets in parallel, the outer diameter of an inlet header of each heat removal tube was 180mm, the outer diameter of an outlet header of each heat removal tube was 180mm, the number of the straight pipes per unit area of the cross section of each heat removal section at the central point thereof was 11.5/m$^2$, each group was formed by connecting 6, 10 and 12 heat removal tube straight pipes in series through straight pipe connecting fittings, the outer diameter of each heat removal tube was 140 mm, and L1/S1 was 5.1. The ratio of the cross-sectional area of the inlet header to the sum of cross-sectional areas of the heat removal branch pipes was 0.83, and the ratio of the cross-sectional area of the outlet header to the sum of cross-sectional areas of the heat removal branch pipes was 0.83.

The feed rate of propylene was 14400 NM$^3$/h, the full propylene treatment capacity at this time was 425kg propylene/h/m$^2$ of the Cross Section A, the reaction temperature was 430 °C, the reaction pressure was 0.04MPa, the propylene: ammonia: air was 1: 1.2: 9.6, the reaction could be stably operated for a long time (such as 10000 h), and the heat quantity removed per hour was 2.40t of 4.5 MPa steam/m$^2$.

**Claims**

1. A heat removal tube set (particularly a heat removal water tube set), **characterized in that** it is configured to be arranged in a heat removal section of a fluidized bed reactor, said heat removal tube set comprising at least 10 (preferably 10 to 100, more preferably 20 to 80) heat removal tubes, said heat removal tube comprising N (N is equal to or greater than 3, preferably N is 3 to 30, more preferably N is 3 to 20) straight pipes and N-1 connecting fittings for connecting any two adjacent straight pipes in series and providing a fluid communication therebetween,

 where the length of the heat removal section along the central axis of the fluidized bed reactor is set as H (in m), a cross section of the heat removal section (referred to as Cross Section A) is obtained by transecting along a direction perpendicular to the central axis of the fluidized bed reactor at a position within the entire region of the length H of the heat removal section (preferably, within the region from 49% H above to 49% H below the central point of the reaction heat removal section, more preferably, within the region from 45% H above to 38% H below the central point of the reaction heat removal section, more preferably, within the region from 40% H above to 8% H below the central point of the reaction heat removal section),

 for at least one (preferably 1, 2 or 3, or at least 20%, at least 50% or at least 65% of the total) of the heat removal tubes (referred to as profiled heat removal tubes) of the heat removal tube set and at most up to 88% (preferably 75% or 70%) of the total of the heat removal tubes of the heat removal tube set, an angle formed between the extended line of the central axis of the projection on the Cross Section A of at least one (preferably at least 2, 3 or 4, and at most 80%, 90% or 100% of the total) of the connecting fittings of the profiled heat removal tube (preferably excluding the first connecting fitting of the profiled heat removal tube) and the extended line of the central axis of the projection on the Cross Section A of at least one other connecting fitting (preferably the other connecting fitting on the profiled heat removal tube at a position immediately upstream of and in fluid communication with said connecting fitting) is greater than 0° and less than 180° (preferably 30°-150°, more preferably 60°-120°, and further preferably about 90°).

2. The heat removal tube set according to claim 1, where the area of the Cross Section A is set as S1 (in $m^2$), and where the sum of the outer contour circumferences of the cross sections of all of the straight pipes of the heat removal tube set on the Cross Section A is set as L1 (in m), L1/S1 is 1.0 to 6.0 $m^{-1}$ (preferably 2.4 to 5.6 $m^{-1}$, more preferably 2.9 to 5.3 $m^{-1}$), and/or the area S1 is 20 to 700 $m^2$ (preferably 35 to 350 $m^2$), and/or the L1 is 20 to 4200 m, preferably 87.5 to 1225 m.

3. The heat removal tube set according to claim 1, where the total number of the straight pipes in the heat removal tube set in the Cross Section A is set as Nt, the number of straight pipes per unit area of the Cross Section A, i.e. Nt/S1, is 4-16/$m^2$ (preferably 5-14/$m^2$, and more preferably 7-13/$m^2$), and/or the profile of the Cross Section A is circular, elliptical or oval, and preferably circular or substantially circular, and/or the inner profile and the outer contour of the cross section of the straight pipe are circular, elliptical or oval, and preferably circular or substantially circular.

4. The heat removal tube set according to claim 1, **characterized in that** the heat removal tube set is capable of recovering 1-10 MPa saturated steam (preferably 2-8 MPa saturated steam, more preferably 3-5 MPa saturated steam), and/or has a heat removal capacity of 0.5-3.0 t saturated steam per unit cross-sectional area ($m^2$) per hour, preferably 1.0-2.8 t saturated steam per unit cross-sectional area ($m^2$) per hour, more preferably 1.2-2.4 t saturated steam per unit cross-sectional area ($m^2$) per hour, when evaluated as recovering 4.5MPa saturated steam, wherein said unit cross-sectional area refers to the unit area of the Cross Section A.

5. The heat removal tube set according to claim 1, wherein for heat removal tube(s) of said heat removal tube set other than said profiled heat removal tube, an angle formed between an extended line of the central axis of the projection of any connecting fitting on said Cross Sectional A and an extended line of the central axis of the projection of another connecting fitting on said heat removal tube at a position immediately upstream or downstream of and in fluid communication with said connecting fitting on said Cross Sectional A is 180°, and/or wherein said heat removal tube comprises a cooling water inlet, and the cooling water inlets of a plurality (preferably 2-8, 2-6 or 2-4) of said heat removal tubes are merged into a cooling water inlet header in said heat removal section, and/or wherein said heat removal tube comprises a cooling water outlet, and the cooling water outlets of a plurality (preferably 2-8, 2-6 or 2-4) of said heat removal tubes are merged into a cooling water outlet header in the heat removal section.

6. The heat removal tube set according to claim 1, **characterized in that** the outer diameters of the straight pipes are respectively 80-180 mm, preferably 90-170 mm, and/or the inner diameters of the straight pipes are respectively 60-150 mm, preferably 70-140 mm, and/or the lengths of the straight pipes are respectively 4-13 m, preferably 5-12.0 m, and/or the spacing between two adjacent straight pipes on each heat removal tube is 100-700 mm, preferably 150-300 mm, and/or the length H of the heat removal section is 4-13 m (preferably 5-12 m).

7. The heat removal tube set according to claim 1, **characterized in that**,

(1) where the full propylene treatment capacity per hour of the fluidized bed reactor is 140-290 kg propylene/$m^2$ of the Cross Section A, excluding the endpoint of 290, L1/S1 is 1.0-2.5 $m^{-1}$ (excluding the endpoint of 2.5), preferably 1.4-2.2 $m^{-1}$, or
(2) where the full propylene treatment capacity per hour of the fluidized bed reactor is 200-370 kg propylene/$m^2$ of the Cross Section A, L1/S1 is 1.8-4.6 $m^{-1}$, preferably 2.0-4.1 $m^{-1}$, or
(3) where the full propylene treatment capacity per hour of the fluidized bed reactor is 290-445 kg propylene/$m^2$ of the Cross Section A, L1/S1 is 2.5-6.0 $m^{-1}$, preferably 2.9-5.3 $m^{-1}$.

8. A fluidized bed reactor, **characterized in that** it comprises a head, a dilute phase zone, a heat removal section, a pre-reaction section and a cone from top to bottom in sequence, wherein a heat removal tube set according to claim 1 is arranged in the heat removal section.

9. A method for producing an unsaturated nitrile, comprising the step of subjecting an olefin (such as propylene) to an ammoxidation reaction in a fluidized bed reactor according to claim 8 to obtain an unsaturated nitrile (such as acrylonitrile).

10. A method for increasing the load of a fluidized bed reactor, wherein the full propylene treatment capacity per hour of the fluidized bed reactor is 140-290 kg propylene/$m^2$ of the Cross Section A (excluding the endpoint of 290) and L1/S1 is 1.0-2.5 $m^{-1}$ (excluding the endpoint of 2.5), the method comprising increasing L1/S1 to 2.5-6.0 $m^{-1}$, preferably to 2.9-5.3 $m^{-1}$, while increasing the full propylene treatment capacity per hour of the fluidized bed reactor to 290-445 kg propylene/$m^2$ of the Cross Section A.

11. A method for producing an unsaturated nitrile, comprising the step of subjecting an olefin (such as propylene) to an ammoxidation reaction in a fluidized bed reactor to obtain an unsaturated nitrile (such as acrylonitrile), wherein the load of the fluidized bed reactor is increased according to the method for increasing the load as described in claim 10.

12. The method according to claim 9 or 11, wherein the molar ratio of propylene/ammonia/air (calculated as molecular oxygen) is 1 : 1.1-1.3 : 1.8-2.0, the reaction temperature is 420-440 °C, the reaction pressure (gauge pressure) is 0.03-0.14 MPa, and the weight hourly space velocity of the catalyst is 0.06-0.15 $h^{-1}$.

Fig. 1

Fig. 2

Coventional heat
removal tube

L

Cross Section A

Profiled heat
removal tube

Fluidized bed reactor

Fig. 3

Fig. 4A

A1

6 6 6

5 5 5

Fig. 4B

5 5 5

6 6 6

A1

Fig. 4C

5 5 5

6 6 6

A 1

Fig. 4D

Fig. 5

Fig. 6

3(4)

3(4)

3(4)

Fig. 7

## INTERNATIONAL SEARCH REPORT

International application No.

**PCT/CN2022/085766**

### A. CLASSIFICATION OF SUBJECT MATTER

B01J 8/24(2006.01)i; B01J 8/00(2006.01)i; C07C 253/18(2006.01)i; C07C 255/08(2006.01)i

According to International Patent Classification (IPC) or to both national classification and IPC

### B. FIELDS SEARCHED

Minimum documentation searched (classification system followed by classification symbols)

B01J;C07C

Documentation searched other than minimum documentation to the extent that such documents are included in the fields searched

Electronic data base consulted during the international search (name of data base and, where practicable, search terms used)

CNABS, CNTXT, SIPOABS, DWPI, CNKI; 撤热管, 换热管, 直管, 竖管, 连接管, 串联, 横切面, 夹角, 不饱和腈, heat withdrawal tube, heat removal pipe, heat exchange tube, straight tube, straight pipe, standpipe, connecting pipe, series, transverse plane, included angle, unsaturated nitrile

### C. DOCUMENTS CONSIDERED TO BE RELEVANT

| Category* | Citation of document, with indication, where appropriate, of the relevant passages | Relevant to claim No. |
|---|---|---|
| X | CN 102010350 A (NINGBO TECHNOLOGY RESEARCH INSTITUTE, CNPC et al.) 13 April 2011 (2011-04-13) description, paragraphs [0009]-[0022], and figures 1-4 | 10-12 |
| Y | CN 102010350 A (NINGBO TECHNOLOGY RESEARCH INSTITUTE, CNPC et al.) 13 April 2011 (2011-04-13) description, paragraphs [0009]-[0022], and figures 1-4 | 1-9 |
| Y | CN 112050469 A (QINGDAO HAIER SMART TECHNOLOGY RESEARCH & DEVELOPMENT CO., LTD. et al.) 08 December 2020 (2020-12-08) description, paragraphs [0004]-[0013], and figure 4 | 1-9 |
| A | CN 112050469 A (QINGDAO HAIER SMART TECHNOLOGY RESEARCH & DEVELOPMENT CO., LTD. et al.) 08 December 2020 (2020-12-08) description, paragraphs [0004]-[0013], and figure 4 | 10-12 |
| Y | CN 109574833 A (CHINA PETROLEUM & CHEMICAL CORPORATION et al.) 05 April 2019 (2019-04-05) description, paragraphs [0007]-[0027], and figure 1 | 1-12 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 June 2022** | **01 July 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/085766**

### C.    DOCUMENTS CONSIDERED TO BE RELEVANT

| Category* | Citation of document, with indication, where appropriate, of the relevant passages | Relevant to claim No. |
|---|---|---|
| A | CN 204051647 U (MAO XUEFENG) 31 December 2014 (2014-12-31)<br>entire document | 1-12 |
| A | CN 101281008 A (UNIVERSITY OF SHANGHAI FOR SCIENCE AND TECHNOLOGY et al.) 08 October 2008 (2008-10-08)<br>entire document | 1-12 |
| A | JP 2019105381 A (ASAHI KASEI CORPORATION) 27 June 2019 (2019-06-27)<br>entire document | 1-12 |

Form PCT/ISA/210 (second sheet) (January 2015)

INTERNATIONAL SEARCH REPORT
Information on patent family members

International application No.

**PCT/CN2022/085766**

| Patent document cited in search report | | | Publication date (day/month/year) | Patent family member(s) | | | Publication date (day/month/year) |
|---|---|---|---|---|---|---|---|
| CN | 102010350 | A | 13 April 2011 | None | | | |
| CN | 112050469 | A | 08 December 2020 | None | | | |
| CN | 109574833 | A | 05 April 2019 | None | | | |
| CN | 204051647 | U | 31 December 2014 | None | | | |
| CN | 101281008 | A | 08 October 2008 | None | | | |
| JP | 2019105381 | A | 27 June 2019 | CN | 208177426 | U | 04 December 2018 |

Form PCT/ISA/210 (patent family annex) (January 2015)